# EUROPEAN PATENT SPECIFICATION

(11) **EP 2 790 628 B1**
(45) Date of publication and mention of the grant of the patent: **31.10.2018**
(21) Application number: 12857764.0
(22) Date of filing: 13.12.2012
(51) Int. Cl.: A61F 13/49, A61F 13/496, A61F 13/15

(54) **DIAPER**
WINDEL
COUCHE-CULOTTE

(30) Priority: 16.12.2011 JP 2011276053
(43) Date of publication of application: 22.10.2014
(73) Proprietor: 3M Innovative Properties Company, St. Paul, MN 55133-3427 (US)
(72) Inventor: ARAI, Nobuharu, Tokyo 158-8583 (JP); KIMURA, Shinji, Tokyo 158-8583 (JP); ROGERS, John J., Saint Paul, Minnesota 55133-3427 (US)
(74) Representative: Vossius & Partner Patentanwälte Rechtsanwälte mbB
(86) International application number: PCT/US2012/069410
(87) International publication number: WO 2013/090519

(56) References cited:
- EP-A1- 0 604 731
- WO-A1-95/34264
- WO-A1-98/55292
- WO-A1-2006/020690
- WO-A2-97/23348
- JP-A- 2009 072 532
- US-A1- 2003 144 643
- US-A1- 2004 219 854
- US-A1- 2005 043 699
- US-A1- 2005 203 479

## Description

### FIELD OF THE INVENTION

The present invention relates to a diaper.

### BACKGROUND OF THE INVENTION

Disposable diapers are generally classified as either pants-type or open-type (flat-type) diapers. However, in either case, an elastic member may be used on a waist portion or leg opening portions from the perspective of preventing shifting or leakage of discharges and the like when wearing. An example of a pants-type disposable diaper using an elastic member is the diaper described in Japanese Published Unexamined Patent Application No. H9-285487.

When the elastic member is constituted by a rubber thread or another elastic body as used conventionally, wrinkles are introduced in the diaper, and the diaper becomes bulkier overall. In addition, the external appearance tends to be degraded by the wrinkles or bulkiness, and gaps may appear when clothing is put on over the diaper, or the act of putting clothing on may become difficult in itself. Furthermore, because the elastic member is pressed firmly against the body in order to prevent shifting or leakage, the diaper may be difficult to remove once it has been worn, and marks from the elastic member or wrinkles are often left on the skin after wearing.

US 2005/0043699 A1 discloses a diaper having an elastically stretchable composite sheet. The composite sheet comprises a breathable stretchy plastic film formed with a plurality of air passages and breathable heat-sealable fibrous nonwoven fabric layers partially bonded to both surfaces of the plastic film and formed with a plurality of gathers rising and falling along surfaces of the nonwoven fabric layers.

JP 2009 072532 A discloses absorbent underpants having composite stretch sections having external layer sheets joined to internal layer sheets by intermittent or continuous joints in the stretching direction of the composite stretch sections and directions intersecting the stretching direction at right angles, and each of the sheets and is formed by forming a plurality of continuously extending gathers across a plurality of elastic members.

US 2005/0203479 A1 discloses a disposable diaper having a body facing sheet and an undergarment facing sheet in the front and rear waist covering panels that are elastically stretchable in a waist-surrounding direction. These sheets are made of nonwoven fabrics and have regions in which these sheets are placed upon each other and the undergarment facing sheet repetitively undulates in the waist-surrounding direction to form a plurality of gathers.

WO 95/34264 A1 discloses a sheet-like composite comprising a multiplicity of elongate strands of resiliently elastic material, and one or more sheets of material bonded along its length or at sheet bonding locations to the elastic strands, some of which sheets have arcuate portions projecting from the elastic strands between those sheet bonding locations.

### SUMMARY

A diaper as recited in the independent claim is provided. The dependent claims define embodiments.

One aspect of the present invention is a diaper having a waist opening portion and a pair of leg opening portions. The diaper further includes an abdominal-side waist portion and a back-side waist portion forming at least a portion of the waist opening portion, and a body portion including an abdominal-side body portion, a back-side body portion, and a crotch portion that is positioned between the abdominal-side body portion and the back-side body portion to form at least a portion of the leg opening portions. In any portion of the diaper except the crotch portion, the diaper is constituted of a laminate including an extensible fiber aggregate and a plurality of elastomeric molded bodies arranged at intervals, and the elastomeric molded bodies have regions that are joined to the extensible fiber aggregate and regions that are separated from the extensible fiber aggregate.

In another aspect, the elastomeric molded body may be a strand-shaped or a film-shaped elastomeric molded body.

The elastomeric molded body is joined to the extensible fiber aggregate by thermal fusion bonds.

The extensible fiber aggregate comprises elastically resilient composite fibers.

In yet another aspect, a portion of a surface of the elastomeric molded body includes a layer having thermal fusion adhesiveness with a surface of the elastically resilient composite fibers.

Because the laminate has an elastic, flat structure overall, pronounced wrinkles are not generated in the diaper and overall bulkiness of the diaper is eliminated by using the laminate in the abovementioned locations of the diaper. Moreover, this laminate is pressed against the skin evenly and fits to the skin well, and despite being slim overall, the basic diaper functions of preventing shifting and leakage are exhibited without problem. In addition, the diaper is excellent in external appearance and the task of putting clothing on over the diaper is simplified. Furthermore, conspicuous marks are not left on the skin because localized pressing of rubber or the like against the skin is prevented and also because there are few wrinkles.

### BRIEF DESCRIPTION OF THE DRAWINGS

FIG. 1A is a perspective view of a diaper according to a first configuration, wherein FIG. 1B is a front elevation view thereof, and FIG. 1C is a rear elevation view thereof.
FIG. 2A is a perspective view of a diaper according to a second configuration, wherein FIG. 2B is a front elevation view thereof, and FIG. 2C is a rear elevation view thereof.
FIG. 3A is a perspective view of a diaper according to a third configuration, wherein FIG. 3B is a front elevation view thereof, and FIG. 3C is a rear elevation view thereof.
FIG. 4 is a perspective view showing an example of an extensible laminate.
FIG. 5 is a perspective view showing another example of an extensible laminate.
FIGS. 6A, 6B, 6C, and 6D are schematic views showing a plurality of elastomeric molded bodies having a film shape (linear) arranged at intervals.
FIGS. 7A and 7B are schematic views showing a plurality of elastomeric molded bodies having a film shape (curvilinear) arranged at intervals and FIGS. 7C and 7D are schematic views showing a plurality of elastomeric molded bodies having a film shape (annular) arranged at intervals.
FIG. 8A is a section view of a core and sheath type elastomeric molded body. FIG. 8B is a section view of a core and sheath type composite fiber that constitutes the fiber aggregate.
FIG. 9 is an illustration demonstrating an example of a method for producing the extensible laminate shown in FIG. 4.
FIGS. 10A, 10B, and 10C are perspective views of diapers according to first, second, and third embodiments of the present invention, wherein an extensible laminate is applied in a diaper according to the first configuration.
FIGS. 11A, 11B, and 11C are perspective views of diapers according to fourth, fifth, and sixth embodiments of the present invention, wherein an extensible laminate is applied in a diaper according to the second configuration.
FIGS. 12A, 12B, 12C, and 12D are perspective views of diapers according to seventh, eight, and ninth embodiments of the present invention, wherein an extensible laminate is applied in a diaper according to the third configuration.
FIG. 13A is an expanded view of the diaper according to the first configuration and FIG. 13B is a perspective view thereof.
FIG. 14A is an expanded view of the diaper according to the second configuration and FIG. 14B is a perspective view thereof.
FIG. 15A is an expanded view of the diaper according to the third configuration and FIG. 15B is a perspective view thereof.
FIG. 16 is a graph demonstrating a strain test for the extensible laminate.

### DETAILED DESCRIPTION

Embodiments of the present invention are described in detail below while referring to the drawings, but the present invention is not limited to the embodiments below. In the description below, same reference numerals are assigned to same or similar portions, and redundant descriptions are omitted.

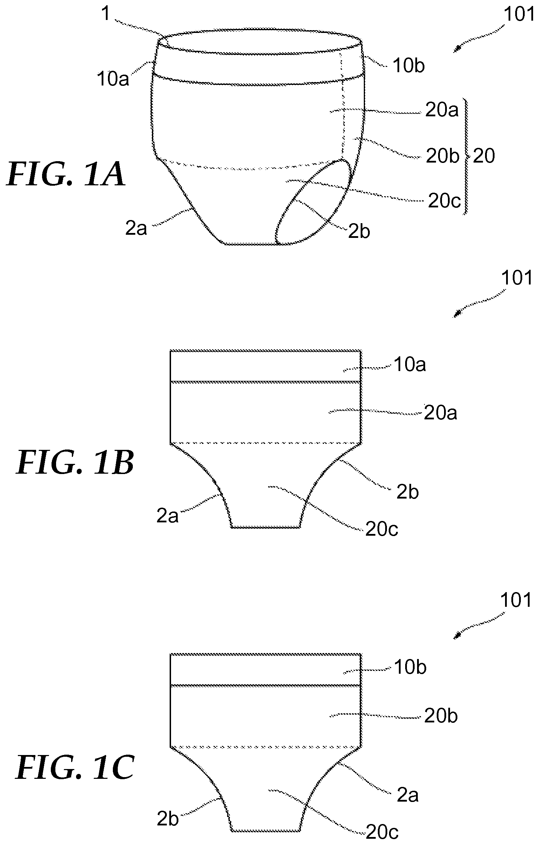

A configuration of a diaper is described first. A diaper that may be included in the present invention is a diaper having a waist opening portion and a pair of leg opening portions, and that is provided with an abdominal-side waist portion, a back-side waist portion, and a body portion. The body portion includes an abdominal-side body portion, a back-side body portion, and a crotch portion that is positioned between the abdominal-side body portion and the back-side body portion to form at least a portion of the leg opening portions. The body portion is adjacent to the abdominal-side waist portion and the back-side waist portion. Any location of the diaper except the crotch portion is constituted by a laminate including an extensible fiber aggregate and a plurality of strand-shaped or film-shaped (linear, curvilinear, annular, or the like) elastomeric molded bodies, which are arranged at intervals. The diaper having such a configuration is described using FIGS. 1 to 3. A diaper that may be included in the present invention is provided with an abdominal-side waist portion, a back-side waist portion, and a body portion as described above. However, this indicates that the diaper includes at least the abdominal-side waist portion, the back-side waist portion, and the body portion, and the diaper may also include other members in addition to the abdominal-side waist portion, the back-side waist portion, and the body portion.

FIG. 1A is a perspective view showing a diaper according to a first configuration, FIG. 1B is a front elevation view, and FIG. 1C is a rear elevation view. A diaper 101 according to the first configuration illustrated in FIG. 1 is a diaper having a waist opening portion 1 and a pair of leg opening portions 2a and 2b, and is constituted by an abdominal-side waist portion 10a and a back-side waist portion 10b, which form the waist opening portion 1, and a body portion 20. The waist opening portion 1 is formed by connecting the abdominal-side waist portion 10a and the back-side waist portion 10b as shown in the perspective view. The body portion 20 includes an abdominal-side body portion 20a, a back-side body portion 20b, and a crotch portion 20c positioned therebetween, and the crotch portion 20c is present between the pair of leg opening portions 2a and 2b. In this manner, the diaper 101 according to the first configuration is a pants-type diaper.

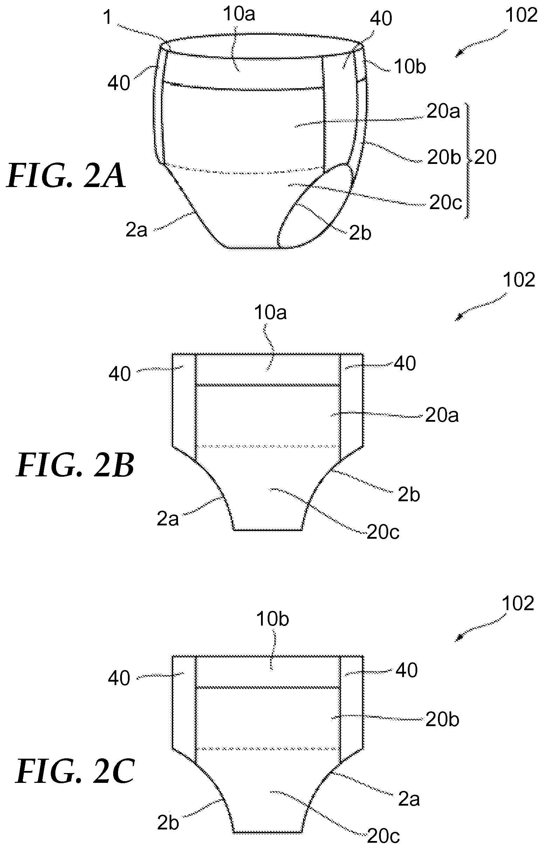

FIG. 2A is a perspective view showing a diaper according to a second configuration, FIG. 2B is a front elevation view, and FIG. 2C is a rear elevation view. A diaper 102 according to the second configuration illustrated in FIG. 2 has a side portion 40 positioned between the abdominal-side waist portion 10a and the back-side waist portion 10b, and the waist opening portion 1 is formed when these portions connect, as shown in the perspective view. The region where the leg opening portions 2a and 2b of the body portion 20 are formed corresponds to the crotch portion 20c, as shown in the front elevation view or the rear elevation view. This crotch portion 20c is positioned between the abdominal-side body portion 20a and the back-side body portion 20b just as in the first configuration. Moreover, the side portion 40 may be made as an extensible portion. In this manner, the diaper 102 according to the second configuration is a pants-type diaper.

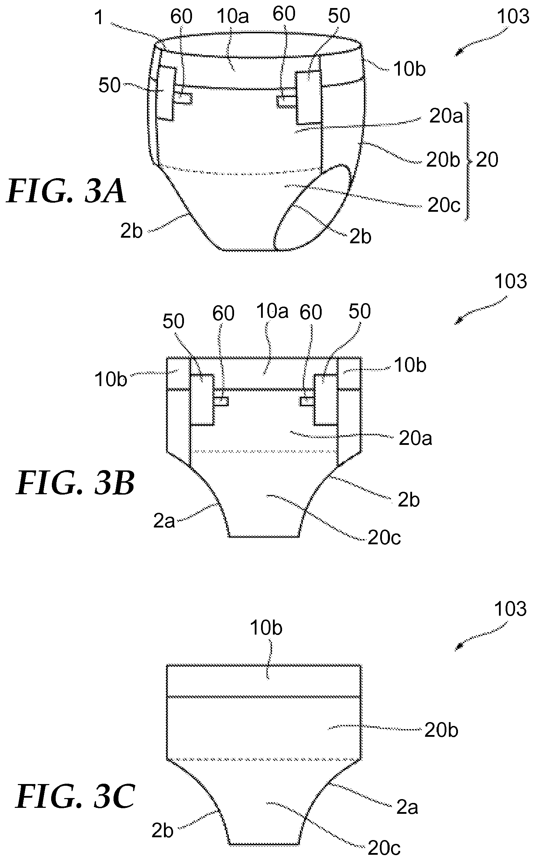

FIG. 3 is an illustration showing a diaper according to a third configuration, and FIG. 3A is a perspective view, FIG. 3B is a front elevation view, and FIG. 3C is a rear elevation view. In a diaper 103 according to the third configuration illustrated in FIG. 3, the waist opening portion 1 is formed by inserting the abdominal-side waist portion 10a and the abdominal-side body portion 20a inside the back-side waist portion 10b and the back-side body portion 20b, and connecting a fastening portion 60, which is joined to an ear portion 50 that is provided on both ends of the back-side waist portion 10b and the back-side body portion 20b, to the abdominal-side body portion 20a in a manner that allows the fastening portion 60 to be detached once again. Moreover, the ear portion 50 may be provided on both ends of either the back-side waist portion 10b or the back-side body portion 20b, and the fastening portion 60 may be connected to the abdominal-side waist portion 10a or to both the abdominal-side waist portion 10a and the abdominal-side body portion 20a in a manner that allows the fastening portion 60 to be detached once again. The shape of the ear portion 50 may be rectangular as illustrated in FIG. 3, and may also be trapezoidal, becoming narrower toward the fastening portion 60. The positions of the crotch portion 20c, abdominal-side body portion 20a, and back-side body portion 20b are the same as in the second configuration. Moreover, the ear portion 50 may be made as an extensible portion. In this manner, the diaper 103 according to the third configuration is an open-type (flat-type) diaper. The fastening portion 60 may be an adhesive portion or a mechanical fastening portion.

Referring to the diaper configurations shown in FIGS. 1 to 3, at least one location of the abdominal-side waist portion 10a, back-side waist portion 10b, abdominal-side body portion 20a, back-side body portion 20b, side portion 40, and ear portion 50 is constituted by a laminate (also called "extensible laminate" hereafter) that includes an extensible fiber aggregate and an elastomeric molded body. In addition to these locations, the crotch portion 20c may also be constituted by an extensible laminate.

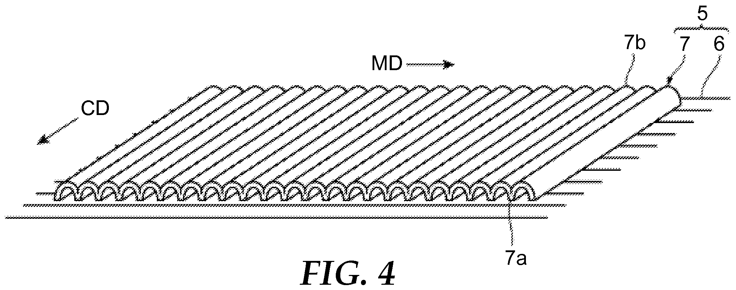

FIG. 4 is a perspective view showing an example of an extensible laminate having a bilayer structure. As shown in FIG. 4, the extensible laminate 5 having a bilayer structure has an extensible fiber aggregate 7 and a plurality of strand-shaped elastomeric molded bodies 6 arranged in parallel in one direction at intervals. The elastomeric molded bodies 6 have regions which are joined to the extensible fiber aggregate 7 and regions which are separated from the extensible fiber aggregate 7, and in the example shown in FIG. 4, the extensible fiber aggregate 7 is formed into a wave shape.

The elastomeric molded bodies 6 are joined to the extensible fiber aggregate 7 by thermal fusion bonding. Materials preferable for joining by thermal fusion bonding will be described below.

A longitudinal direction of the extensible laminate 5 shown in FIG. 4 is treated as the MD (Machine Direction), and a width direction is treated as the CD (Cross Machine Direction). MD refers to a feeding direction of the extensible laminate 5 during production of the extensible laminate 5, and CD refers to a width direction of the extensible laminate 5 orthogonal to MD.

As shown in FIG. 4, a plurality of the strand-shaped elastomeric molded bodies 6 extend along the longitudinal direction (MD), arranged at intervals in the width direction (CD). On the other hand, a fiber aggregate 7 formed into a wave shape has regions that are joined to the elastomeric molded bodies 6 (valley portions 7a) and regions that are separated from the elastomeric molded bodies 6 (arch-shaped ridge portions 7b), alternatingly formed in the longitudinal direction (MD). The valley portions 7a and the ridge portions 7b are formed so as to extend along the width direction (CD). The valley portions 7a and the ridge portions 7b are formed so as to extend along the width direction (CD). The valley portions 7a are joined to the elastomeric molded bodies 6 in a linear formation extending in the width direction (CD). The shape of the ridge portions 7b is not limited to an arch-like shape as viewed in the width direction (CD). For example, the ridge portions 7b may have a square or triangular shape when viewed in the width direction (CD).

According to the extensible laminate 5, an elastic force generated when the extensible laminate 5 is extended in the longitudinal direction (MD) can be varied in two stages. Specifically, in a case with the extensible laminate 5 extended in the longitudinal direction (MD), the elastic force is not sufficiently exhibited until the ridge portions 7b where the elastic fiber aggregate 7 is bent away from the elastomeric molded bodies 6 are extended and flattened. Therefore, in this first stage, the extensible laminate 5 can be extended with a light degree of force that exceeds the elastic force of the elastomeric molded bodies 6. Furthermore, once the ridge portions 7b are extended until flattened, the elastic force of the elastic fiber aggregate 7 is added to the elastic force of the elastomeric molded bodies 6, and the extensible laminate 5 can no longer be extended by a force equivalent to the force that extended and flattened the ridge portions 7b.

Accordingly, with a diaper using the extensible laminate 5, the opening portion can be easily expanded with a mild force to a size at which the diaper can be worn comfortably, and a shape at which the diaper can be worn comfortably can be easily maintained, without the diaper losing shape, by exhibiting a sufficient elastic force after the size at which the diaper can be worn comfortably has been reached. Moreover, with a diaper using the extensible laminate 5, wrinkles can be made inconspicuous by using the fiber aggregate 7 having the valley portions 7a and the ridge portions 7b as a surface. Furthermore, a pattern formed by the valley portions 7a and ridge portions 7b makes it possible to obtain an excellent visual appearance that gives the impression of an undergarment.

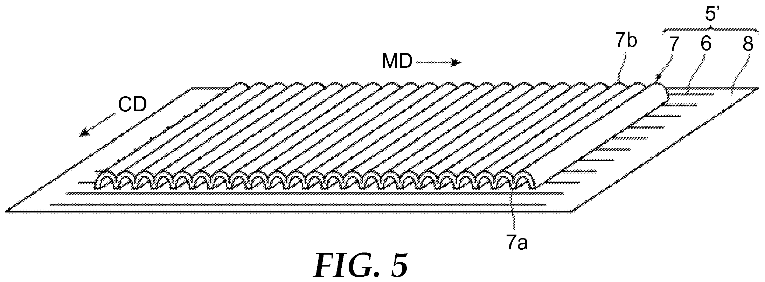

FIG. 5 is a perspective view showing an example of an extensible laminate with a trilayer structure. As shown in FIG. 5, an extensible laminate 5' of a trilayer structure has a plurality of strand-shaped elastomeric molded bodies 6 arranged in parallel in the longitudinal direction (MD) at intervals, an extensible fiber aggregate 7 formed into a wave shape on the elastomeric molded bodies 6, and an elastic fiber aggregate 8 positioned on the opposite side of the fiber aggregate 7 with respect to the elastomeric molded bodies 6. In the extensible laminate 5' having a trilayer structure, the fiber aggregate 7 side is preferably used as a surface side of the diaper, and the fiber aggregate 8 side is preferably used as a user side of the diaper.

The elastomeric molded bodies 6 have regions that are joined to the extensible fiber aggregate 7 and regions that are separated from the extensible fiber aggregate 7. Specifically, the elastomeric molded bodies 6 have regions where a surface facing the fiber aggregate 7 is joined to the valley portions 7a of the fiber aggregate 7 and regions where the surface is separated from the ridge portions 7b of the fiber aggregate 7. That is, some portions of the surface of the elastomeric molded bodies 6 facing the fiber aggregate 7 are not joined to the fiber aggregate 7.

On the other hand, the elastomeric molded bodies 6 may be joined to the flat fiber aggregate 8 across an entire surface. That is, the entire surface of the elastomeric molded bodies 6 facing the fiber aggregate 8 may be joined to the fiber aggregate 8.

The elastomeric molded bodies 6 are joined to the extensible fiber aggregates 7 and 8 by thermal fusion bonding. Materials preferable for joining by thermal fusion bonding will be described below.

By providing the extensible laminate 5' with a trilayer structure having elastic fiber aggregates 7 and 8 on each side of the elastomeric molded bodies 6, an inner side of the diaper that contacts the skin can be formed as a flat fiber aggregate 8. Therefore, the binding pressure is better dispersed, rubber marks are less likely to be left, and fewer pronounced marks are left on the skin after the diaper is removed when worn.

In FIG. 4 and FIG. 5, it is preferable for a pitch of the wave-shaped fiber aggregate 7 (number of ridge portions 7b per 1 cm in the width direction) to be 0.39 cm⁻¹ or more and 11.8 cm⁻¹ or less. A preferred difference between the heights of the lower ends of the valley portions 7a and the upper ends of the ridge portions 7b is from 0.1 mm or more to 5 mm or less. On the other hand, a preferred width of the ridge portions 7b is within a range from 0.1 mm or more to 5 mm or less.

In addition to the strand-shaped elastomeric molded bodies 6 as described above, the elastomeric molded bodies may also be provided as film-shaped. Film-shaped elastomeric molded bodies include linear, curvilinear, and annular elastomeric molded bodies.

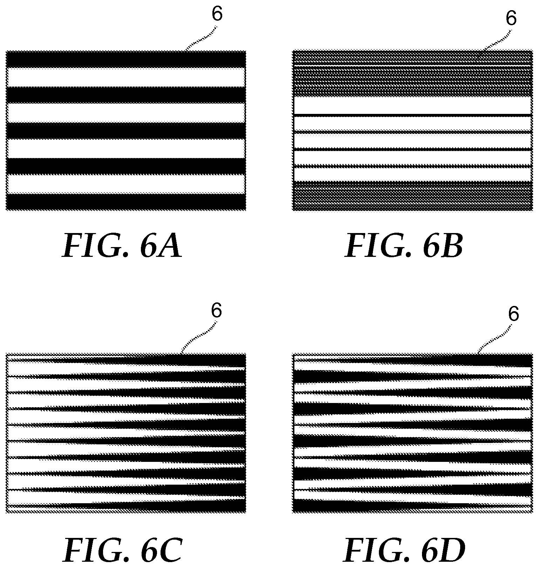

FIGS. 6A, 6B, 6C, and 6D are schematic views of examples of a plurality of elastomeric molded bodies having a film shape (linear) arranged in intervals, in the extensible laminate. A plurality of rectangular elastomeric molded bodies 6 having similar shapes may be arranged at constant intervals as illustrated in FIG. 6A, or a plurality of rectangular elastomeric molded bodies 6 may be arranged at varying intervals so that portions where the elastomeric molded bodies 6 are densely present and portions where elastomeric molded bodies 6 are sparsely present are formed as illustrated in FIG. 6B. The elastomeric molded body 6 may have a linear shape in which a width gradually changes in the longitudinal direction, and a plurality thereof may be arranged so that directions in which the width changes are identical, as illustrated in FIG. 6C, or the elastomeric molded body 6 may have a linear shape in which the width gradually changes in the longitudinal direction, and a plurality thereof may be arranged so that the directions in which the width changes are alternately different, as illustrated in FIG. 6D.

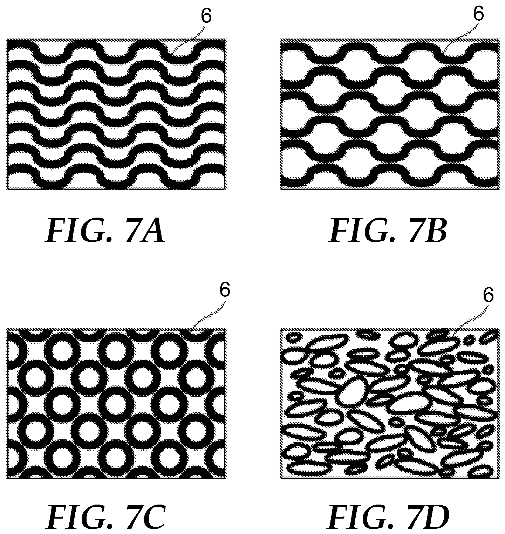

FIGS. 7A and 7B are schematic views of examples of a plurality of elastomeric molded bodies having a film shape (curvilinear) arranged at intervals in the extensible laminate. A plurality of elastomeric molded bodies 6 may be arranged so that the curvilinear shapes align as illustrated in FIG. 7A, or a plurality thereof may be arranged so that the curvilinear shapes face each other as illustrated in FIG. 7B.

FIGS. 7C and 7D are schematic views of examples of a plurality of elastomeric molded bodies having a film shape (annular) arranged at intervals, in the extensible laminate. In this case, annular elastomeric molded bodies 6 having similar shapes may be arranged uniformly at a prescribed interval as illustrated in FIG. 7C, or annular elastomeric molded bodies 6 having different shapes may be arranged uniformly at varying spaces as illustrated in FIG. 7D. The elastomeric molded bodies 6 have the shape, arrangement, and structure as illustrated in FIG. 6 and FIG. 7, for example, and an extensible laminate that is excellent in air-permeability can be obtained thereby.

Regarding dimensions of the strand-shaped or film-shaped elastomeric molded bodies 6 arranged at intervals, when strand-shaped, a preferred diameter of a cross-section of the elastomeric molded body is from 0.01 mm or more to 3 mm or less. On the other hand, for a film shape, in the case of a linear, curvilinear, or an annular film, a preferred width of the elastomeric molded body is 1 mm or more, more preferably 2.5 mm or more, even more preferably 3 mm or more, and even more preferably 5 mm or more. When the dimensions of the strand-shaped or film-shaped elastomeric molded bodies 6 are within this range, and the intervals and arrangement of the elastomeric molded bodies 6 are adjusted, the binding pressure is better dispersed, which makes it less likely for rubber marks to be left behind and results in fewer pronounced marks left on the skin when the diaper is removed when worn. Additionally, a plurality of elastomeric molded bodies 6 spaced apart from each other may be crosslinked with a film material lacking elasticity, to the extent that the aforementioned performance is not impaired. On the other hand, when considering the width of the waist portion and the like, an upper limit for the width of the film-shaped elastomeric molded bodies 6 is normally 30 mm or less, and in certain aspects, preferably 25 mm or less, and more preferably 20 mm or less. In addition, the interval between adjacent elastomeric molded bodies 6 can be set from 1 mm or more to 10 mm or less. The air-permeability tends to degrade when the interval is narrower than 1 mm, and the binding pressure tends to weaken when the interval is wider than 10 mm. From this perspective, the preferred interval is 2 mm or more and 5 mm or less.

Methods for forming the plurality of film-shaped elastomeric molded bodies 6 arranged at intervals include, for example, a method in which a cylinder is engraved with a pattern corresponding to the elastomeric molded bodies 6 as illustrated in FIGS. 6 and 7, a molten elastomer is poured into the cylinder, and then the elastomer is transferred onto the fiber aggregate 7; and a method in which a pattern corresponding to a portion other than the elastomeric molded bodies 6 illustrated in FIGS. 6 and 7 is die cut from the elastomeric molded bodies, and the elastomeric molded bodies 6 are affixed to the fiber aggregate 7 after die cutting.

An elastomeric molded body 6 consisting of a material having adjustable elasticity is preferred, and a thermoplastic elastomer is particularly preferable as such a material. The thermoplastic elastomer is generally constituted by a soft component having molecular based rubber elasticity (soft segment, soft phase) and a molecular constraining component (hard segment, hard phase) for preventing plastic deformation, and the thermoplastic elastomer can be classified according to the type of this hard segment. As the thermoplastic elastomer for the elastomeric molded body 6, (1) urethane-based thermoplastic elastomers (TPU), (2) ester-based thermoplastic elastomers, (3) olefin-based thermoplastic elastomers (TPO), (4) styrene-based thermoplastic elastomers, (5) vinyl chloride-based thermoplastic elastomers, (6) amide-based thermoplastic elastomers, (7) syndiotactic poly(1,2-butadiene), (8) poly(trans-1,4-isoprene), and similar polymers can be preferably used.

Among these, (1) urethane-based thermoplastic elastomers, (2) ester-based thermoplastic elastomers, (3) olefin-based thermoplastic elastomers, (4) styrene-based thermoplastic elastomers, and combinations thereof are preferable, (1) urethane-based thermoplastic elastomers, (2) ester-based thermoplastic elastomers, (4) styrene-based thermoplastic elastomers, and combinations thereof are more preferable; and (1) urethane-based thermoplastic elastomers, (4) styrene-based thermoplastic elastomers, and combinations thereof are even more preferable.
(1) Examples of urethane-based thermoplastic elastomers generally include thermoplastic elastomers having intramolecular urethane bonds, obtained by bringing about a polyaddition reaction of a long-chain polyol, short-chain polyol, or other polyol component with a diisocyanate or other isocyanate.
(1) As polyols that can be used as a source composition of the urethane-based thermoplastic elastomers, polyester-based (adipate-based, polycaprolactone-based, and the like), and polyether-based polyols are typical. Examples of long-chain polyols include polyether diols (for example, poly(oxytetramethylene) glycol and poly(oxypropylene) glycol), polyester diols (for example, poly(ethylene adipate) glycol, poly(1,4-butylene adipate) glycol, poly(1,6-hexylene adipate) glycol, and poly (hexanediol 1,6-carbonate) glycol), and the like. Examples of short-chain diols include ethylene glycol, 1,3-propylene glycol, bisphenol-A, 1,4-butanediol, 1,4-hexanediol, and the like.

Examples of diisocyanates can also include any aromatic, aliphatic, or cycloaliphatic isocyanate, such as 4,4'-diphenylmethane diisocyanate, toluene diisocyanate, hexamethylene diisocyanate, and the like.

In certain aspects, a Shore A hardness (JIS A hardness) of the (1) urethane-based thermoplastic elastomer can be from 60 or more to 95 or less. When the Shore A hardness (JIS A hardness) of the (1) urethane-based thermoplastic elastomer is within a range from 60 or more to 95 or less, the stability of a film when the elastomer composition is melted and formed into a film can be increased, and a film having favorable elastic flexibility can be obtained. In certain aspects, two or more kinds of the urethane-based thermoplastic elastomers may be used in combination.

Examples of elastomers that are preferable for use as the (2) ester-based thermoplastic elastomers include ester-based elastomers containing a block having an aromatic polyester as the hard segment and a block having an aliphatic polyether or an aliphatic polyester as the soft segment, and the like.

Use of an ethylene-α-olefin copolymer prepared using metallocene as a catalyst for the (3) olefin-based thermoplastic elastomer is particularly preferable, especially considering processability, cost, light resistance, chemical resistance, skin irritation, and the like. Examples of α-olefins copolymerized with ethylene in the ethylene-α-olefin copolymer include α-olefins having from 3 to 30 carbon atoms, for example, propylene, 1-butene, 1-pentene, 1-hexene, 1-octene, 1-heptene, 4-methyl-1-pentene, 4-methyl-1-hexene, 4,4-dimethyl-1-pentene, octadecene, and the like. Among these, the use of 1-hexene, 1-octene, 1-heptene, and 4-methyl-1-pentene is preferred. A preferred mixture ratio of ethylene and α-olefin in the ethylene-α-olefin copolymer is from 40 wt.% or more to 98 wt.% or less of ethylene and from 2 wt.% or more to 60 wt.% or less of α-olefin.

Specific examples of the (4) styrene-based thermoplastic elastomers that can be used include various types of tertiary block polymer materials having aromatic vinyl-conjugated diene (or one in which a portion or all of the unsaturated bonds thereof are hydrogenated)-aromatic vinyl block copolymers as basic structures. Styrene is desirable as the vinyl monomer constituting the aromatic vinyl polymer. Moreover, isoprene is desirable as the monomer constituting the conjugated diene. A part or all of the unsaturated bonds thereof may be hydrogenated at the time when used as a styrene-based thermoplastic elastomer. Representative examples of the (4) styrene-based thermoplastic elastomers include styreneisoprene-styrene block copolymers (SIS copolymers).

In the case when an SIS copolymer is used as the (4) styrene-based thermoplastic elastomer, a preferred proportion of styrene is 10 wt.% or more, and more preferably 15 wt.% or more, and is preferably 50 wt.% or less, and particularly preferably 45 wt.% or less, when an entire weight of the SIS copolymer is taken as 100 wt.%.

A high melt flow rate (200°C, 5.0 kg) of the SIS copolymer with respect to fluidity (processability) and film stability when the source composition of the elastomeric molded body 6 is rendered into a lamellar form is preferred, and the melt flow rate can be set from 10 g/10 min or more to 45 g/10 min or less in certain aspects. In addition, in certain aspects, a lower limit of the melt flow rate of the SIS copolymer can be set at 20 g/10 min and a higher limit can be set at 40 g/10 min.

Unmodified types and modified types of SIS copolymers can be used. A modified SIS copolymer can be obtained by, for example, bringing about an addition reaction (for example, grafting) of an unsaturated carboxylic acid or derivative thereof on an SIS copolymer. Specific examples include maleic acid, fumaric acid, itaconic acid, acrylic acid, crotonic acid, endo-bi-cyclo-[2,2,1]-5-heptene-2,3-dicarboxylic acid, cis-4-cyclohexene-1,2-dicarboxylic acid, and anhydrides and imides thereof.

In certain aspects, an SIS copolymer having a skeleton with three or more branches can be used as the SIS copolymer. Moreover, in certain aspects, two or more kinds of SIS copolymers may be used in combination.

Tackifiers (tackifying agents) and other additives may be included in addition to these polymer components in the source composition of the elastomeric molded body 6.

Tackifiers having good compatibility with the polymers are preferred. When a blended polymer of the (1) urethane-based thermoplastic elastomer and the (4) styrene-based thermoplastic elastomer (for example an SIS copolymer) is used as the polymer component, a blended polymer that does not damage the structure of the urethane-based thermoplastic elastomer and that has good compatibility with the styrene-based thermoplastic elastomer is preferred. As the tackifier, rosin-based, terpene-based, petroleum-based tackifiers, and the like can be used.

In certain aspects, the softening point of the tackifier can be set to a range of from 40°C or more to 160°C or less, or from 70°C or more to 160°C or less. Also, in certain aspects, two or more kinds of tackifiers may be used in combination.

A quantity of tackifier can be set to from 0.1 wt.% or more to 10 wt.% or less based on a total weight of the source composition of the elastomeric molded body 6.

The source composition of the elastomeric molded body 6 may further include various kinds of additives (antioxidants, weathering agents, UV absorbers, colorants, inorganic fillers, oils, and the like). For example, thermoplastic plastics, oil components, or the like, may be added in order to modify the melt fluidity of the thermoplastic elastomers.

From a perspective of elasticity of the laminate, a preferred basis weight of the film-shaped elastomeric molded body 6 is 300 g/m² or lower, more preferably 200 g/m² or lower, and even more preferably 100 g/m² or lower. Basis weight as shown here indicates a degree to which the elastomeric molded body 6 is present, relative to a surface area of the entire laminate. On the other hand, from the perspective of durability, the basis weight of the elastomeric molded body 6 is preferably 5 g/m² or more, and more preferably 10 g/m² or more. Moreover, a preferred arrangement in the width direction (CD) of the extensible laminate of the strand-shaped elastomeric molded bodies 6 is from 1 strand/cm or more to 20 strands/cm or less.

The film-shaped elastomeric molded body 6 may be a single-layer structure or a multilayer structure. In the case of a multiple-layer structure, each layer may be constituted with a different elastomeric molded body. In that case, at least one layer of the plurality of layers is constituted by a thermoplastic elastomer as mentioned above. From the perspective of elasticity, a preferred overall thickness of the elastomeric molded body 6 is 300 µm or less, and more preferably 200 µm or less. On the other hand, from the perspective of durability, the preferred overall thickness of the elastomeric molded body 6 is 20 µm or more, and more preferably 30 µm or more. The elastomeric molded body 6 may have a uniform thickness or a varying thickness. A thickness per sheet of elastomeric molded body 6 can be set from about 5 µm or more to about 100 µm or less. A surface of the film-shaped elastomeric molded body 6 may be provided with a layer having thermal fusion adhesiveness with a surface of the fiber aggregates 7 and 8.

The strand-shaped elastomeric molded body 6 may use a composite fiber having a strand form such as a parallel type (side-by-side type), a split type (fiber cross section split into arc shapes), a core and sheath type (concentric and eccentric type), or the like.

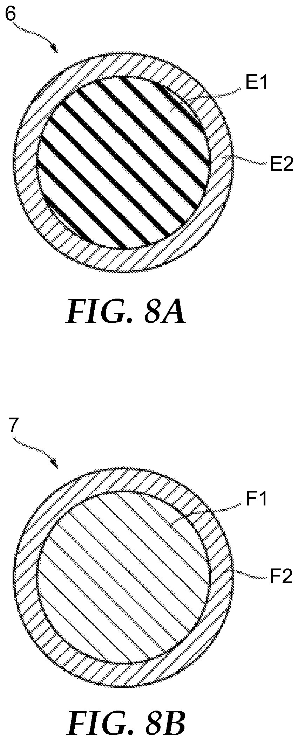

FIG. 8A shows an example of a strand-shaped elastomeric molded body 6 in the form of a core and sheath type. The strand-shaped elastomeric molded body 6 has a core material E1 and a sheath material E2 that covers the core material E1.

The thermoplastic elastomers described above as materials to be used for the elastomeric molded body 6 can be used as the core material E1, but of these elastomers, styrene-based thermoplastic elastomers are preferred. An example of a styrene-based thermoplastic elastomer is the (4) styrene-based thermoplastic elastomer described above. The proportion of styrene in the styrene-based thermoplastic elastomer, the melt flow rate in the case of an SIS copolymer, the potential to use the native/denatured states, the presence or absence of a branched structure, as well as the presence or absence, content, and types of additives such as tackifiers are as described above.

A material having thermal fusion adhesiveness with respect to the fiber aggregate 7 is preferred as the sheath material E2. As shown in FIG. 8B, if the fiber aggregate 7 includes a core and sheath type composite fiber containing a core material F1 and a sheath material F2 which covers the core material F1, the use of a material with thermal fusion adhesiveness to the sheath material F2 as the sheath material E2 is preferred. Thermal fusion bonding of the core and sheath type composite fiber shown in FIG. 8B, the elastomeric molded body 6, and the fiber aggregate 7 will be described below.

A non-elastomeric component can be used as the sheath material E2 that exhibits thermal fusion adhesiveness to the sheath material F2. Examples of non-elastomeric components include olefin-based resins such as polyethylene, polypropylene, and the like, or polyester-based resins such as polyethylene terephthalate (PET), polybutylene terephthalate (PBT), and the like. A crystalline or non-crystalline component may be used for the non-elastomeric component. Examples of polyethylenes include low-density polyethylene (LDPE), linear low-density polyethylene (LLDPE), and high-density polyethylene (HDPE), and examples of polypropylenes include propylene homopolymers, propylene-based binary copolymers, and propylene-based ternary copolymers. From the perspective of thermal fusion adhesiveness, the use of crystalline polypropylene is preferred.

The crystalline polypropylene can be used with no particular limitation provided that it has hard elasticity. Preferable examples of the crystalline polypropylene include homopolymers of propylene, copolymers mainly of propylene with ethylene, copolymers mainly of propylene with α-olefins, and the like.

A preferred crystallinity of the crystalline polypropylene is 40% or more. The elastic recovery of the fiber may be insufficient when the crystallinity is less than 40%. The crystallinity is a value calculated based on the energy required to melt the crystal as measured by DSC (differential scanning calorimetry).

A preferred melt index of the crystalline polypropylene is from 1 g/10 min or more to 200 g/10 min or less, and is more preferably from 3 g/10 min or more to 50 g/10 min or less. When the melt index is less than 1 g/10 min, the melt viscosity becomes too high and spinning may become difficult in some cases. When the melt index exceeds 200 g/10 min, the melt viscosity becomes too low and thread breakage may occur in some cases before fibers are formed. The preferred melt index is therefore within the aforementioned range. The melt index is a value measured at 230°C under a load of 2.16 kgf following ASTM D-1238.

A preferred weight average molecular weight of the crystalline polypropylene is from 10,000 or more to 1,000,000 or less, and is more preferably from 20,000 or more to 600,000 or less, with respect to an elastic resiliency that is readily expressed.

A preferred ratio of the cross-sectional areas of the core material E1 and the sheath material E2 is in a range from 50:50 to 99.9:0.1.

In this way, because the core and sheath type elastomeric molded body 6 has a sheath material E2 which is thermally adhesive to the sheath material F2, it possible to realize a strong bond between the elastomeric molded body 6 and the fiber aggregate 7 by thermal fusion bonding. Moreover, since the elastomeric molded body 6 and the fiber aggregate 7 can be firmly joined, it is possible to eliminate the need for an adhesive at the time of the manufacture of the extensible laminates 5 and 5'.

There is no particular limitation to the fiber aggregate 7 and fiber aggregate 8, provided that these are extensible and air-permeable with a pleasant tactile feel, and examples include nonwovens constituted by monofilament or composite fibers having these characteristics. The fibers constituting the fiber aggregates 7 and 8 will be described in detail hereinafter. The fibers constituting the fiber aggregates 7 and 8 may be the same or different.

Specific examples of the fibers constituting the fiber aggregates include (i) olefin-based, polyester-based, polyamide-based, and other synthetic fibers of polyethylene, polypropylene, or the like, (ii) regenerated fibers such as rayon, cupra, and the like, and natural fibers such as cotton and the like, two-component elastically resilient composite fibers that use a non-elastomeric component as described above as a material for the sheath material E2 and particularly a hard elastic component made from the crystalline polypropylene described above as a first component and a thermoplastic elastomer as described above as a material for the elastomeric molded body 6 as a second component, and (iii) mixed spinning fibers formed by mixing these fibers.

These fibers may be processed by a spunbond process, spunlace process, thermal bond process, melt-blowing process, needle-punching process, or other suitable processes to obtain the fiber aggregates 7 and 8.

Examples include spunbond nonwovens subjected to a drawing process to a prescribed stretch ratio after production, nonwovens subjected to a blade-groove drawing process, low-interlace spunlace, pleat-processed nonwovens, and the like. From the perspective of elongation under low load and from the aspect of material strength, use of a spunbond nonwoven subjected to a drawing process to a prescribed stretch ratio after production is particularly preferred.

A nonwoven obtained by various processes for production of nonwovens including elastic fibers may also be used as the fiber aggregates 7 and 8. The fiber aggregate may also be constituted with identical or different inelastic fiber layers, which are substantially inelastic, laminated on both sides of an elastic fiber layer.

The fiber aggregate may also be in a state in which a portion of the constituent fiber of at least one of the two inelastic fiber layers is inserted into the elastic fiber layer, and/or a state in which a portion of the constituent fiber of the elastic fiber layer is inserted into at least one of the inelastic fiber layers. Being in such a state promotes integration between the elastic fiber layer and the inelastic fiber layers and effectively prevents the occurrence of floating between both layers.

The elastic fiber layer is an aggregate of a fiber having elasticity. Examples of processes for forming the fiber having elasticity include a melt-blowing process whereby a molten resin is extruded from nozzle pores and the extruded resin in the molten state is extended by hot blast to form narrow fibers, and a spun bond process whereby resin in a semi-molten state is drawn by cold air or mechanical drawing. There is also a spinning-blowing process combining a melt-blowing process and spunbond process as a special method for a melt-blowing process. In addition, the elastic fiber layer may be in the form of a web or nonwoven made from fiber having elasticity. For example, the elastic fiber layer may be a web or nonwoven formed by a spinning-blowing process, spunbond process, melt-blowing process, or the like. A web obtained by a spinning-blowing process is particularly preferred.

Examples of fibers that can be used for constituting the elastic fiber layer include, for example as described above for the elastomeric molded body 6, fibers having thermoplastic elastomers, rubber, and the like, as source materials. Fibers having thermoplastic elastomers as source materials are particularly optimal for the extensible fiber aggregate of the present embodiment that uses air-through nonwoven as the basic structure because melt-spinning using an extruder is possible just as with ordinary thermoplastic resins, and because the fibers thus obtained are easily fused thermally. Examples of thermoplastic elastomers include SBS, SIS, SEBS, SEPS, and other styrene-based thermoplastic elastomers, olefin-based thermoplastic elastomers, ester-based thermoplastic elastomers, urethane-based thermoplastic elastomers, and the like. These may be used singly or in combinations of two or more kinds.

The inelastic fiber layer has extensibility but is substantially inelastic. "Extensibility" here may be either a case in which the constituent fibers themselves extend, or a case in which the constituent fibers themselves do not extend, but rather pairs of thermally fused fibers move apart at a point of intersection between the fibers, the three-dimensional structure formed by the plurality of fibers changes structurally due to the thermal fusion between the fibers, or the constituent fibers tear apart and the overall fiber layer thereby extends.

Examples of fibers constituting the inelastic fiber layer include fibers containing polyethylene (PE), polypropylene (PP), polyester (PET or PBT), polyamide, and the like. The fibers constituting the inelastic fiber layer may be short fibers or long fibers, and may also be hydrophilic or hydrophobic. Core and sheath type or side-by-side composite fiber, split fiber, irregular cross section fiber, crinkled fiber, and heat shrink fiber and the like may also be used. These fibers may be used singly or in combinations of two or more kinds. The inelastic fiber layer may be a continuous filament, a short fiber web, or a nonwoven. A short fiber web is particularly preferable because a bulky inelastic fiber layer having thickness can be formed. Two inelastic fiber layers may be the same or may be different in relation to material of the constituent fiber, basis weight, thickness, and the like. In the case of a core and sheath type composite fiber, the core is preferably PET or PP and the sheath is preferably low-melting-point PET, PP, or PE. Use of these composite fibers is particularly preferable, from aspects of thermal fusion bonding with the constituent fiber of the elastic fiber layer containing a styrene-based elastomer becoming stronger and peeling between layers being less likely to occur.

A two-component-type elastically resilient composite fiber (hereinafter referred to simply as "composite fiber"), having an elastically resilient composite fiber as described above, in other words, having a non-elastomeric component (particularly a hard elastic component) as a first component and having a thermoplastic elastomer as a second component, can be used as the fiber constituting the fiber aggregate from the perspective of imparting extensibility to the fiber aggregate. A fiber aggregate constituted by this composite fiber is cloth-like to the touch just as an ordinary nonwoven, and has excellent texture. Accordingly, a diaper having an extensible laminate including this fiber aggregate feels comfortable when worn.

As an example of a non-elastomeric component that can be used as the first component of the composite fiber, a component the same as the sheath material E2 described above can be included. From the perspective of thermal fusion adhesiveness, use of the crystalline polypropylene as described above is preferred. The preferred weight average molecular weight of the crystalline polypropylene is from 10,000 or more to 1,000,000 or less, and is more preferably from 20,000 or more to 600,000 or less, due to the aspects that elastic resiliency is readily expressed and the composite fiber is easily spun.

The second component of the composite fiber includes a thermoplastic elastomer. The thermoplastic elastomers described above as materials to be used for the elastomeric molded body 6 can be used as this thermoplastic elastomer.

An elastic recovery percentage at 100% elongation of the thermoplastic elastomer of 50% or more is preferable, due to the aspect that the extensible laminates 5 and 5' will be capable of following bodily movements without breaking.

In the composite fiber, a preferred content of the first component is 5 wt.% or more and 70 wt.% or less, and a preferred content of the second component is 30 wt.% or more and 95 wt.% or less. A more preferred content of the first component is 10 wt.% or more and 60 wt.% or less, and a more preferred content of the second component is 40 wt.% or more and 90 wt.% or less. Even more preferably, the content of the first component is 10 wt.% or more and 50 wt.% or less, and the content of the second component is 50 wt.% or more and 90 wt.% or less. When the content of the first component exceeds this upper limit or the content of the second component is less than this lower limit, the elasticity of the composite fiber may be insufficient in some cases. When the content of the first component is less than the lower limit or the content of the second component exceeds the upper limit, an area where the second component is exposed on the surface of the composite fiber becomes greater and the feel may be degraded in some cases, and it also may become difficult to spin a core and sheath type composite fiber in some cases. Therefore, setting the contents within the above ranges is preferred.

There is no particular limitation to a fiber form of the composite fiber provided that the fiber form is such that the composite fiber is capable of expressing elastic resiliency. Examples of preferable fiber forms of the composite fiber include parallel types (side-by-side types), split types (fiber cross-section split into arc forms), core and sheath types (concentric types and eccentric types), and the like. In the case of a core and sheath type, a first component is used as the sheath material and a second component is used as the core material.

As shown in FIG. 8B, the core and sheath type composite fiber contains a core material F1 and a sheath material F2 which covers the core material F1. A preferred ratio of cross-sectional areas of the core material F1 and the sheath material F2 in the core and sheath type composite fiber is within a range from 50:50 to 99.9:0.1.

As the core material F1, the (1) urethane-based thermoplastic elastomer or the (4) styrene-based thermoplastic elastomer described above as materials to be used for the elastomeric molded bodies 6 may be used. Of these, the (1) urethane-based thermoplastic elastomer is preferable. The same is true for the polyol component and isocyanate constituting the urethane-based thermoplastic elastomer as described above and the Shore A hardness. The proportion of styrene in the styrene-based thermoplastic elastomer, the melt flow rate in the case of an SIS copolymer, the potential to use the native/denatured states, the presence or absence of a branched structure, as well as the presence or absence, content, and types of additives such as tackifiers are as described above.

An example of the sheath material F2 is a non-elastomeric component as described above as a material to be used for the sheath material E2. The preferred component constituting the sheath material F2 is a polymer of the same type as the sheath material E2 from the perspective of thermal adhesiveness to the core and sheath type elastomeric molded body 6.

In this way, since the core and sheath type composite fiber has a sheath material F2 which has thermal fusion adhesiveness to the sheath material E2, it is possible to realize a strong bond between the elastomeric molded body 6 and the fiber aggregate 7 by thermal fusion bonding. Moreover, since the elastomeric molded body 6 and the fiber aggregate 7 can be firmly joined, it is possible to eliminate the need for an adhesive at the time of the manufacture of the extensible laminates 5 and 5'.

The composite fiber can be produced by a publicly-known spinning process. The composite fiber produced by the publicly-known spinning process may be prepared into a web immediately after spinning, and a nonwoven may then be formed. Alternatively, from an aspect of further expressing elastic characteristics, the composite fiber may be prepared into a web after undergoing a prescribed drawing treatment after spinning, and a nonwoven may then be formed. Preferable conditions for the drawing treatment are a drawing temperature from 20°C or more to 130°C or less and a stretch factor of 1 times or more and 6 times or less. Examples of means that can be used for heating the elastically resilient composite fiber in the drawing treatment include hot blast, steam, infrared radiation, and the like.

The fiber diameter of the composite fiber is preferably 1 denier or more to 20 denier or less, and is more preferably 2 denier or more to 6 denier or less. When the fiber diameter is less than 1 denier, spinnability in the spinning process is degraded and fiber formation may become difficult. When the fiber diameter is greater than 20 denier, the texture may be degraded for practical utility of the elastically resilient nonwoven. The fiber diameter is therefore preferably within these ranges.

A preferred elastic recovery percentage at 100% elongation of the composite fiber is from 20% or more to 100% or less, and is more preferably from 50% or more to 100% or less. When the elastic recovery percentage is less than 20%, the function of following bodily movements of the elastic side panel may be insufficient.

The composite fiber may be used in the form of short fiber such as staple fiber, or may be used in the form of long fiber such as continuous filament.

The fiber aggregate 7 may be constituted 100% by the composite fiber, but may also be mixed with other fibers. In the case when the composite fiber is mixed with another fiber, the elastically resilient nonwoven preferably contains 30 wt.% or more of the composite fiber, and more preferably contains 50 wt.% or more. The elastically resilient nonwoven becomes markedly lowered in elastic resiliency and may break when the content of the composite fiber is less than 30 wt.%. Other fibers that can be mixed with the composite fiber are fibers that are not deteriorated by thermal treatment in the process of formation of the nonwoven and include for example polyolefin, polyester, polyamide, and other thermoplastic synthetic fibers; cotton, flax/hemp, wool, and other natural fibers; rayon, acetate, and other regenerated fibers; various kinds of binder fibers that can be fused by the thermal treatment and the like.

The fiber aggregate 7 can be produced, for example, by a process using a carding machine or by a direct sheet process. Specifically, the fiber aggregate can be produced in the form of a nonwoven by resin bonding, mixing with binder fiber, heated roll, water needling, and other processes for production of nonwovens. In particular, the fiber aggregate 7 is preferably produced by forming a web composed of the composite fiber (or including the composite fiber) by a publicly-known web formation process, then fusing between the points of interlacing of the fibers in the web by heating at a temperature between the melting point of the first component and the melting point of the second component, and forming a large number of contact points. An example of a process for forming the web, in the case when staple fiber or another short fiber is used as the composite fiber, is a process in which the composite fiber is spread open using a carding machine and a web is formed. Also, an example in the case when continuous filament or other long fiber is used as the composite fiber is a process in which the melt-spun composite fiber is conveyed on a high-speed air flow and is deposited and spread open on a moving net and a web is formed (spunbond process).

An example of a process for heating the formed web and forming a nonwoven (thermal bonding process) is a process in which the web is passed through a through-air dryer, the constituent fibers of the web are thermally fused between the points of interlacing by hot blast, and several contact points are formed. In this case, a temperature of the hot blast and the quantity supplied depend on the kind of constituent fiber of the web, the basis weight of the web, and the speed of conveyance and the like, but in general, the preferred temperature of the hot blast is from 140°C or more to 170°C or less, and a preferred flow speed or wind speed is from 0.5 m/min to 3 m/min. An example of another process for the thermal treatment is a hot embossing process using a pair of embossing rolls including an engraved roll and a smooth roll. In this case, hot embossing processing is performed by heating either or both of these rolls. A preferred heating temperature of the embossing rolls is from 120°C or more to 170°C or less. The web may join to the embossing rolls when the embossing rolls are heated to a temperature higher than this. Examples of the engraved roll include iron rolls engraved on the surface with various patterns. On the other hand, examples of the smooth roll include paper rolls, rubber rolls, silicone rubber rolls, urethane rubber rolls, metal rolls, and the like. Examples of a pattern on the engraved roll include pins, dots, tortoise shells, lattices, longitudinal stripes, lateral stripes, stitching meshes, and designs, but there is no particular limitation to the pattern. A linear pressure of the embossing rolls during the thermal embossing process depends on the basis weight of the web, the speed of conveyance, and the heating temperature of the embossing rolls, but from 10 kg/cm or more to 150 kg/cm or less is preferable as a general range.

A preferred elastic recovery percentage at 20% elongation of the fiber aggregate 7 is from 40% or more to 100% or less, and is more preferably from 60% or more to 100% or less. When the elastic recovery percentage is less than 40%, the following of bodily movements and the like of the extensible laminate may be insufficient and resistance may become greater.

The fiber aggregate 7 can be used respectively with a thickness of about 200 µm or less. 150 µm or less is preferable, and 80 µm or less is more preferable so that the bulk is not increased and the feeling is not degraded while sufficient elasticity is still provided. On the other hand, about 30 µm or more is preferable, and 35 µm or more is more preferable, from the perspective of durability. Also, the fiber aggregate 7 can be used with a fabric weight from 1 g/m² or more to 500 g/m² or less, but from a perspective of ease of production, 400 g/m² or less is preferable, and 300 g/m² or less is more preferable. Moreover, 200 g/m² or less is particularly preferable. On the other hand, 3 g/m² or more is preferable, and 5 g/m² or more is more preferable, from a perspective of durability.

The joining of the elastomeric molded body 6 and the elastically resilient composite fiber in the laminate is explained here. For joining of the elastomeric molded body 6 and the elastically resilient composite fiber, joining using an adhesive material, joining by thermal fusion bonding, and the like may be used.

In joining using an adhesive material, the adhesive material is used in the form of a spray or a coating for example. The type of adhesive material is not particularly restricted, and olefin based, natural rubber based, urethane based, acrylic based, and silicone based adhesive materials, and the like can be used.

In thermal fusion bonding, for example, bonding together is performed at from 200 to 300°C (melt laminating). When carrying out joining by performing thermal fusion bonding, it is preferable that the material on the surface of the elastomeric molded bodies 6 and the material on the surface of the fiber constituting the fiber aggregates 7 and 8 have same or similar chemical structures. The chemical structure being the same or similar means that, for example, common structures are present in at least a part of the monomers forming the source compositions. Examples of such relationships include PP and PP (same), PP and PE (similar), and the like (in the above example, the carbon - carbon double bond is common). Further, it is preferable that the fibers constituting the fiber aggregates 7 and 8 are elastically resilient composite fibers. In addition, a layer having thermal fusion adhesiveness with the surface of the elastically resilient composite fiber may be present on a part of the surface of the elastomeric molded body 6. By selecting such materials, it is possible to join the elastomeric molded body 6 to the fiber aggregates 7 and 8 by thermal fusion bonding.

Apart from this, it is also possible to join the elastomeric molded body 6 and the elastically resilient composite fiber by a physical method such as knitting, stitching, and the like.

### Production Method of Extensible Laminate

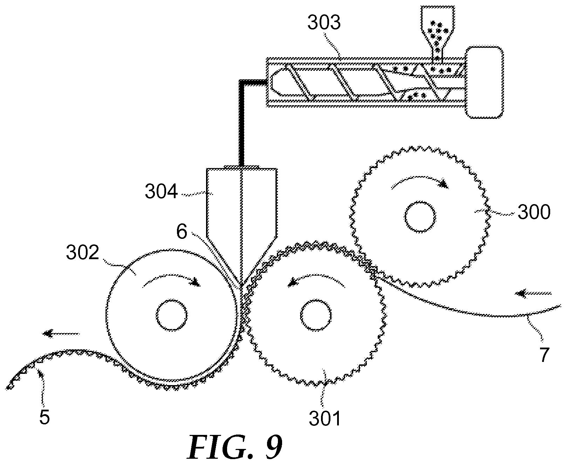

In the following, referring to FIG. 9, an example is described of a method of producing an extensible laminate 5 with a bilayer structure. First, a sheet-shaped fiber aggregate 7 is produced using a melt-blowing process. The produced sheet-shaped fiber aggregate 7 is fed, as indicated by the arrow in FIG. 9, between forming rolls 300 and 301 having wave-shaped undulating patterns, thereby forming a wave-shaped fiber aggregate 7 having valley portions 7a and ridge portions 7b. By changing the rolls 300 and 301 used, it is possible to change the shape of the fiber aggregate 7 (the shapes of the valley portions 7a and ridge portions 7b) to any desired shape. The fiber aggregate 7 formed in the shape of a wave is fed between the forming roll 301 and a chilling roll 302 by the rotation of the forming roll 301.

Meanwhile, plasticizing of the elastomer is carried out in an extruder 303, and the plasticized elastomer is pushed out by the extruder 303 and fed to a T die 304. The elastomer is formed into a plurality of strand-shaped elastomeric molded bodies 6 by passing through the T die 304. The elastomeric molded bodies 6 pushed out in a molten state from the T die 304 are supplied to the area between the forming roll 301 and the chilling roll 302. After that, an extensible laminate 5 with a bilayer structure is produced between the forming roll 301 and the chilling roll 302, by joining the fiber aggregate 7 formed in the shape of a wave and the plurality of elastomeric molded bodies 6.

It is possible to use an adhesive material or to use thermal fusion bonding for joining of the fiber aggregate 7 and the elastomeric molded bodies 6. When using thermal fusion bonding, it is possible to increase the strength of bonding by making the surface of the elastomeric molded bodies 6 and the fiber surface of the fiber aggregate 7 a material having thermal fusion adhesiveness as described above.

Further, when producing an extensible laminate 5' with a trilayer structure, it is possible to obtain the extensible laminate 5' with a trilayer structure by further feeding a fiber aggregate 8 between the forming roll 301 and the chilling roll 302.

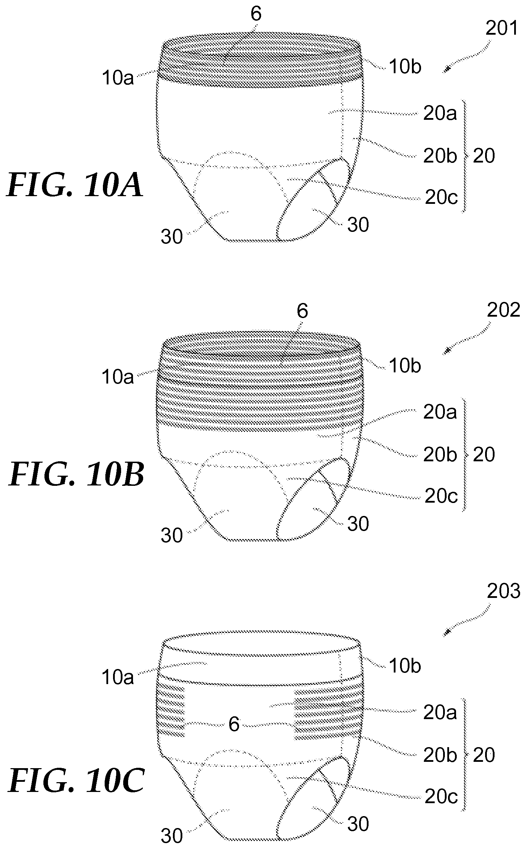

Diapers according to embodiments of the present invention are described next using FIGS. 10 to 12. FIGS. 10A, 10B, and 10C are perspective views of diapers according to first, second, and third embodiments of the present invention, using the extensible laminates 5 and 5' described above in diapers according to the first configuration. FIG. 10A is a perspective view of the diaper 201 according to the first embodiment. An extensible laminate 5 or 5' is present on an abdominal-side waist portion 10a and a back-side waist portion 10b. Specifically, in FIG. 10A, the horizontal-striped line area (thick black lines) on the abdominal-side waist portion 10a and the back-side waist portion 10b indicates the film-shaped (linear) elastomeric molded bodies 6 arranged at intervals, as described above. In the case when the elastomeric molded bodies 6 are present on the main surfaces of the extensible fiber aggregates 7, the abdominal-side waist portion 10a and the back-side waist portion 10b are constituted by extensible laminate 5 in the form illustrated in FIG. 4. In addition, in the case when the elastomeric molded bodies 6 are arranged between opposed extensible fiber aggregates 7 and 8, the abdominal-side waist portion 10a and the back-side waist portion 10b are constituted by extensible laminate 5' in the form illustrated in FIG. 5. An absorbent body 30 is fixed to a crotch portion 20c. Note that in all aspects of FIG. 10, the elastomeric molded bodies 6 are indicated with thick black lines for ease of understanding, but in the case when the extensible laminate 5 is used, the elastomeric molded bodies 6 may be present on fiber aggregates 7 on the inside or on the outside of the diaper. In addition, in the case when the extensible laminate 5' is used, the elastomeric molded bodies 6 are present between the fiber aggregates 7 and 8. This arrangement of such elastomeric molded bodies 6 is also the same in the second to tenth embodiments below.

FIG. 10B is a perspective view of the diaper 202 according to the second embodiment. An extensible laminate 5 or 5' is present on an abdominal-side waist portion 10a, a back-side waist portion 10b, an abdominal-side body portion 20a, and a back-side body portion 20b. Specifically, in FIG. 10B, the horizontal-striped line area on the abdominal-side waist portion 10a, back-side waist portion 10b, abdominal-side body portion 20a, and back-side body portion 20b indicates the plurality of film-shaped (linear) elastomeric molded bodies 6 arranged at intervals as described above. The abdominal-side waist portion 10a, back-side waist portion 10b, abdominal-side body portion 20a, and back-side body portion 20b are constituted by the extensible laminate 5 in the form illustrated in FIG. 4, or by the extensible laminate 5' in the form illustrated in FIG. 5. An absorbent body 30 is fixed to a crotch portion 20c.

FIG. 10C is a perspective view of the diaper 203 according to the third embodiment. An extensible laminate 5 or 5' is present on the abdominal-side body portion 20a and the back-side body portion 20b. Specifically, in FIG. 10C, the horizontal-striped line area on the abdominal-side body portion 20a and the back-side body portion 20b indicates the plurality of film-shaped (linear) elastomeric molded bodies 6 arranged at intervals as described above. The abdominal-side body portion 20a and the back-side body portion 20b are constituted by the extensible laminate 5 in the form illustrated in FIG. 4 or the extensible laminate 5' in the form illustrated in FIG. 5. An absorbent body 30 is fixed to a crotch portion 20c.

The diaper according to the first configuration is a pants-type diaper in which a waist opening portion 1 is formed by connecting the abdominal-side waist portion 10a and the back-side waist portion 10b. A diaper having a further eliminated bulkiness and having a more compact configuration can be provided through the presence of the extensible laminates 5 or 5' on each location of the diaper.

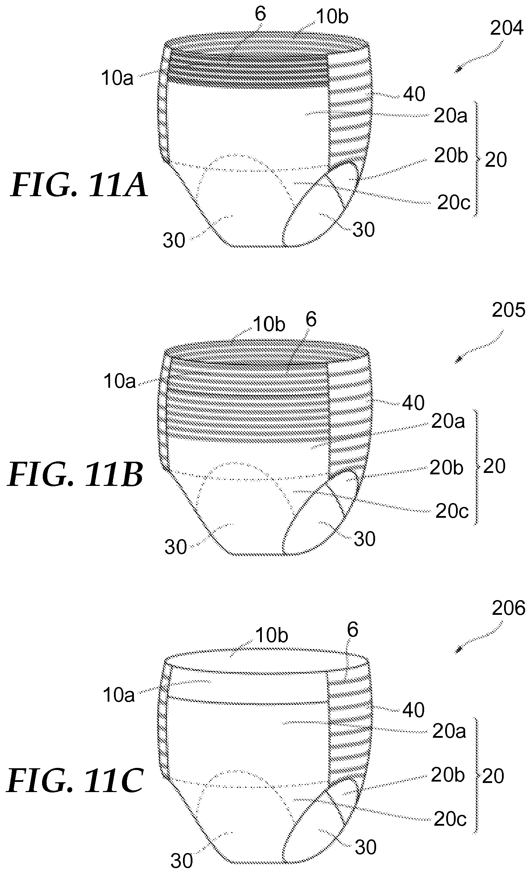

FIGS. 11A, 11B, and 11C are perspective views of diapers according to fourth, fifth, and sixth embodiments of the present invention, using the extensible laminates 5 or 5' described above in diapers according to the second configuration. FIG. 11A is a perspective view of the diaper 204 according to the fourth embodiment. An extensible laminate 5 or 5' is present on an abdominal-side waist portion 10a, a back-side waist portion 10b, and a side portion 40. Specifically, in FIG. 11A, the horizontal-striped line area on the abdominal-side waist portion 10a, back-side waist portion 10b, and the side portion 40 indicates the plurality of film-shaped (linear) elastomeric molded bodies 6 arranged at intervals as described above. The abdominal-side waist portion 10a, back-side waist portion 10b, and side portion 40 are constituted by the extensible laminate 5 in the form illustrated in FIG. 4, or by the extensible laminate 5' in the form illustrated in FIG. 5. An absorbent body 30 is fixed to a crotch portion 20c.

FIG. 11B is a perspective view of the diaper 205 according to the fifth embodiment. An extensible laminate 5 or 5' is present on an abdominal-side waist portion 10a, a back-side waist portion 10b, a side portion 40, an abdominal-side body portion 20a, and a back-side body portion 20b. Specifically, in FIG. 11B, the horizontal-striped line area on the abdominal-side waist portion 10a, back-side waist portion 10b, side portion 40, abdominal-side body portion 20a, and back-side body portion 20b indicates the plurality of film-shaped (linear) elastomeric molded bodies 6 arranged at intervals as described above. The abdominal-side waist portion 10a, back-side waist portion 10b, side portion 40, abdominal-side body portion 20a, and back-side body portion 20b are constituted by the extensible laminate 5 in the form illustrated in FIG. 4, or by the extensible laminate 5' in the form illustrated in FIG. 5. An absorbent body 30 is fixed to a crotch portion 20c.

FIG. 11C is a perspective view of the diaper 206 according to the sixth embodiment, and an extensible laminate 5 or 5' is present on a side portion 40. Specifically, in FIG. 11C, the horizontal-striped line area on the side portion 40 indicates the plurality of film-shaped (linear) elastomeric molded bodies 6 arranged at intervals as described above. The side portion 40 is constituted by the extensible laminate 5 in the form illustrated in FIG. 4, or by the extensible laminate 5' in the form illustrated in FIG. 5. An absorbent body 30 is fixed to a crotch portion 20c.

The diaper according to the second configuration is a pants-type diaper in which a waist opening portion 1 is formed by connecting the abdominal-side waist portion 10a, back-side waist portion 10b, and side portion 40. A diaper having a further eliminated bulkiness and having a more compact configuration can be provided through the presence of the extensible laminates 5 or 5' on each location of the diaper. Moreover, the sense of fit to the body can be further improved by the presence of the extensible laminates 5 or 5' on the side portion 40.

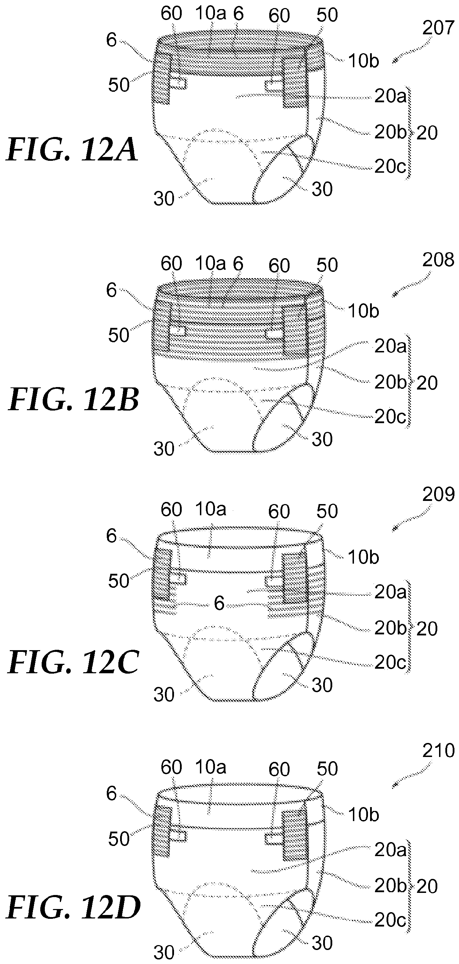

FIGS. 12A, 12B, 12C, and 12D are perspective views of diapers according to seventh, eighth, ninth, and tenth embodiments of the present invention, using the extensible laminates 5 and 5' as described above in diapers according to the third configuration. FIG. 12A is a perspective view of the diaper 207 according to the seventh embodiment, and an extensible laminate 5 or 5' is present on an abdominal-side waist portion 10a, a back-side waist portion 10b, and an ear portion 50. Specifically, in FIG. 12A, the horizontal-striped line area on the abdominal-side waist portion 10a, back-side waist portion 10b, and ear portion 50 indicates the plurality of film-shaped (linear) elastomeric molded bodies 6 arranged at intervals as described above. The abdominal-side waist portion 10a, back-side waist portion 10b, and ear portion 50 are constituted by the extensible laminate 5 in the form illustrated in FIG. 4, or by the extensible laminate 5' in the form illustrated in FIG. 5. An absorbent body 30 is fixed to a crotch portion 20c.

FIG. 12B is a perspective view of the diaper 208 according to the eighth embodiment. An extensible laminate 5 or 5' is present on an abdominal-side waist portion 10a, a back-side waist portion 10b, an ear portion 50, an abdominal-side body portion 20a, and a back-side body portion 20b. Specifically, in FIG. 12B, the horizontal-striped line area on the abdominal-side waist portion 10a, back-side waist portion 10b, ear portion 50, abdominal-side body portion 20a, and back-side body portion 20b indicates the plurality of film-shaped (linear) elastomeric molded bodies 6 arranged at intervals as described above. The abdominal-side waist portion 10a, back-side waist portion 10b, ear portion 50, abdominal-side body portion 20a, and back-side body portion 20b are constituted by the extensible laminate 5 in the form illustrated in FIG. 4, or by the extensible laminate 5' in the form illustrated in FIG. 5. An absorbent body 30 is fixed to a crotch portion 20c.

FIG. 12C is a perspective view of the diaper 209 according to the ninth embodiment, and an extensible laminate 5 or 5' is present on an abdominal-side body portion 20a, a back-side body portion 20b, and an ear portion 50. Specifically, in FIG. 12C, the horizontal-striped line area on the abdominal-side body portion 20a, back-side body portion 20b, and ear portion 50 indicates the plurality of film-shaped (linear) elastomeric molded bodies 6 arranged at intervals as described above. The abdominal-side body portion 20a, back-side body portion 20b, and ear portion 50 are constituted by the extensible laminate 5 in the form illustrated in FIG. 4, or by the extensible laminate 5' in the form illustrated in FIG. 5. An absorbent body 30 is fixed to a crotch portion 20c.

FIG. 12D is a perspective view of the diaper 210 according to the tenth embodiment, and an extensible laminate 5 or 5' is present on an ear portion 50. Specifically, in FIG. 12D, the horizontal-striped line area on the ear portion 50 indicates the plurality of film-shaped (linear) elastomeric molded bodies 6 arranged at intervals as described above. The ear portion 50 is constituted by the extensible laminate 5 in the form illustrated in FIG. 4, or by the extensible laminate 5' in the form illustrated in FIG. 5. An absorbent body 30 is fixed to a crotch portion 20c.

The diaper according to the third configuration is an open-type diaper in which a waist opening portion 1 is formed by connecting a fastening portion 60 joined with the ear portion 50 to the abdominal-side body portion 20a in a manner that allows the fastening portion 60 to be detached once again. In the diaper according to the third configuration, the abdominal-side waist portion 10a and the abdominal-side body portion 20a are inserted inside the back-side waist portion 10b and the back-side body portion 20b. Mutually overlapping portions therefore arise between the ends of the abdominal-side waist portion 10a and abdominal-side body portion 20a and the ends of the back-side waist portion 10b and back-side body portion 20b. The configuration would therefore normally tend to become bulky. However, with the diaper according to the present embodiment, a diaper having a further eliminated bulkiness and having a more compact configuration can be provided despite being an open-type diaper. The effect is particularly great in the eighth embodiment illustrated in FIG. 12B, in which the extensible laminates are used in all locations of the abdominal-side waist portion 10a, abdominal-side body portion 20a, back-side waist portion 10b, and back-side body portion 20b.

The present invention is not limited to the diapers according to the embodiments illustrated in FIGS. 10 to 12, and the same kind of effect can be obtained, provided that an extensible laminate 5 or 5' is used in at least one location of the abdominal-side waist portion 10a, back-side waist portion 10b, abdominal-side body portion 20a, back-side body portion 20b, side portion 40, and ear portion 60. Moreover, it is not necessary that the extensible laminate be formed across an entire region respectively of each location of the diaper, and it is sufficient that the extensible laminate 5 or 5' be formed on at least a portion thereof.

### Process for Preparation of the Diaper

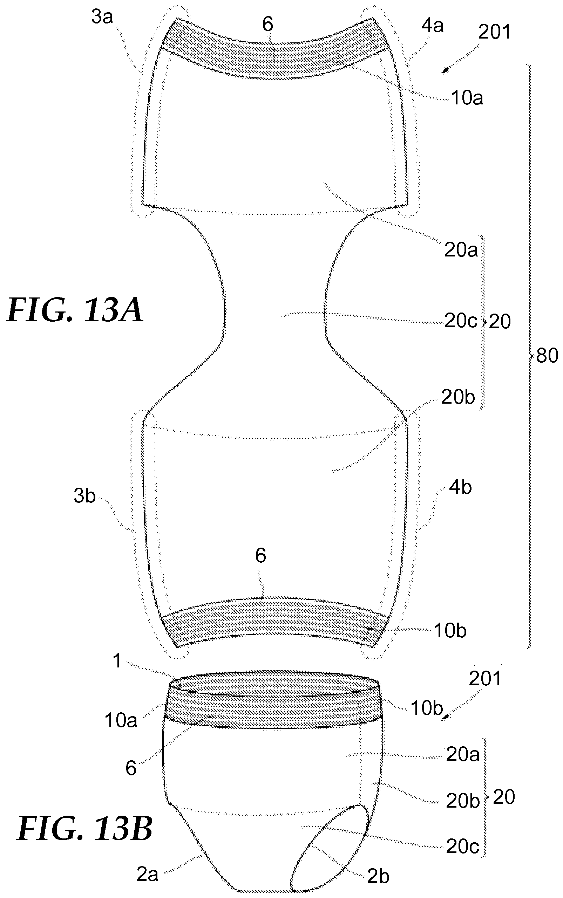

Processes for preparation of the diapers according to the first embodiment, fourth embodiment, and seventh embodiment are described below while referring to FIGS. 13 to 15.

In the process for preparation of the diaper 201 according to the first embodiment, for example, a sheet-shaped outer body containing an extensible laminate 5 or 5' and an inner body that serves as a surface used when wearing are respectively produced on separate lines, and the diaper 201 is then produced on one line after the outer body and inner body have undergone an integration step. The production process includes, for example, a step for formation of an outer body, a step for formation of a crotch portion, a step for attachment of an inner body, a step for joining side portions, and a step for cutting away a waist portion.

The step for formation of an outer body is a step for forming an outer portion to constitute a diaper body 80 including an abdominal-side waist portion 10a, a back-side waist portion 10b, and a body portion 20. In the case when the entirety of the outer portion constituting the diaper body 80 is formed with one kind of fiber aggregate 7 and fiber aggregate 8, specifically, an elastomeric molded body 6 is provided on the fiber aggregate 7 and fiber aggregate 8 and two sheets of fiber aggregate 7 and fiber aggregate 8 are affixed together with the elastomeric molded body 6 interposed in between. In a location where the fiber aggregate 7 is not provided, a sheet-shaped outer body is continuously formed by providing a rubber thread or other elastic member on the fiber aggregate 7 and fiber aggregate 8 or by successively affixing together two sheets of fiber aggregate 7 and fiber aggregate 8 using a hot-melt adhesive, or the like, while sandwiching a rubber thread or other elastic member between the two sheets of fiber aggregate 7 and fiber aggregate 8.

The step for formation of a crotch portion is a step for inserting a curved cut in the sheet-shaped outer body in the center of the sheet-shaped outer body so that a narrow part is formed on both sides as illustrated in FIG. 13A. Leg opening portions 2a and 2b as illustrated in FIG. 13B are formed during formation of the diaper or when putting the diaper on, by the narrow part formed by this step.

The step for attachment of an inner body is a step for attaching an inner body having an absorbent body 30 to the outer body for example. For example, the inner body is affixed by hot-melt adhesive, or the like, on the surface of the sheet-shaped outer body so as to cover the narrow part formed in the step for formation of a crotch portion.

The step for joining side portions is a step for folding the diaper body 80 at the center of the crotch portion 20c so that the abdominal-side waist portion 10a and back-side waist portion 10b as well as the abdominal-side body portion 20a and back-side body portion 20b respectively overlap, joining a left side 3a of the abdominal-side waist portion 10a and abdominal-side body portion 20a and a left side 3b of the back-side waist portion 10b and back-side body portion 20b using a thermal fusion bonding or hot-melt adhesive, and joining a right side 4a of the abdominal-side waist portion 10a and abdominal-side body portion 20a and a right side 4a of the back-side waist portion 10b and back-side body portion 20b using a thermal fusion bonding or hot-melt adhesive. A waist opening portion 1 and leg opening portions 2a and 2b are thereby formed, and a pants-type diaper as illustrated in FIG. 13B is assembled.

The step for cutting away a waist portion is a step for cutting a remainder of the formed sheet-shaped outer body from the ends of the abdominal-side waist portion 10a and the back-side waist portion 10b. The diaper 201 according to the first embodiment is produced by the above steps.

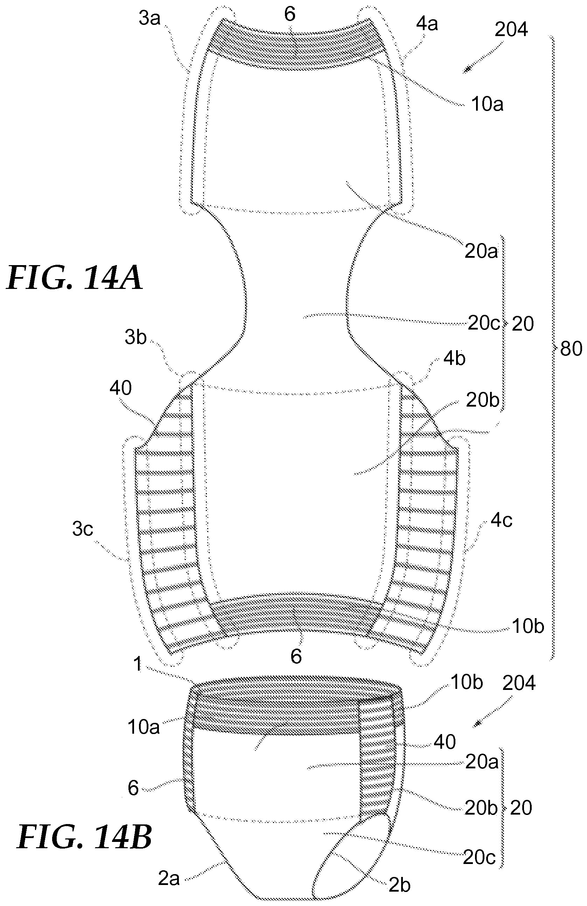

In the diaper 204 according to the fourth embodiment, extensible side portions 40 are joined respectively with a left side 3b and a right side 4b of the back-side waist portion 10b and the back-side body portion 20b as illustrated in FIGS. 14A and 14B. An extensible side portion 40 may be formed by providing the elastomeric molded body 6 described above, a rubber thread, or other elastic member on the fiber aggregate 7 constituting the diaper body 80; or an extensible side portion may be formed by providing the elastomeric molded body 6 described above, a rubber thread, or other elastic member on a fiber aggregate 7 other than the fiber aggregate 7 constituting the diaper body 80, and joining the other fiber aggregate with the back-side waist portion 10b and the back-side body portion 20b using an adhesive.

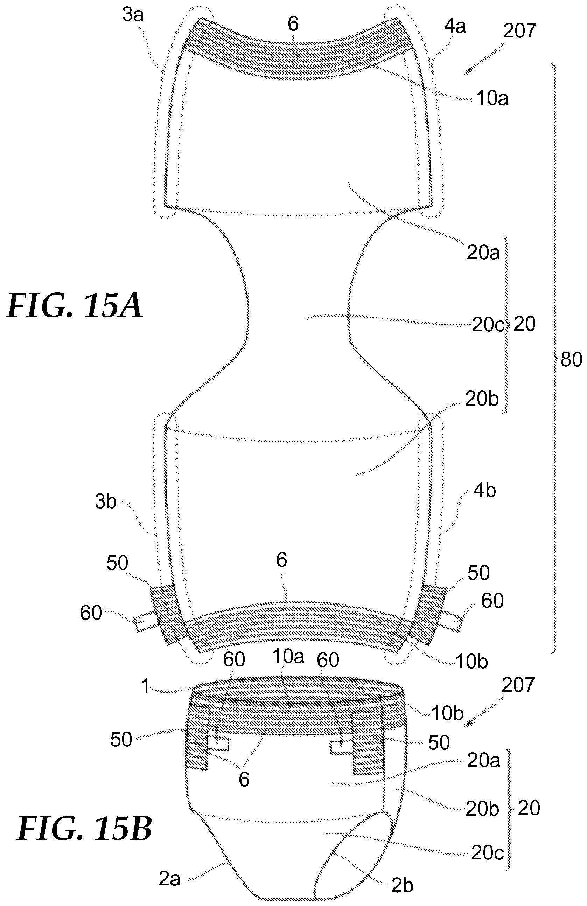

In the diaper 207 according to the seventh embodiment, an extensible ear portion 50 having an adhesive or mechanical fastening portion 60 on the front end is joined respectively with a left side 3b and a right side 4b of the back-side waist portion 10b and the back-side body portion 20b as illustrated in FIGS. 15A and 15B. The extensible ear portion 50 may be formed by providing the elastomeric molded body 6 described above, a rubber thread, or other elastic member on the fiber aggregate 7 constituting the diaper body 80; or the extensible ear portion may be formed by providing the elastomeric molded body 6 described above, a rubber thread, or other elastic member on a fiber aggregate 7 other than the fiber aggregate 7 constituting the diaper body 80, and joining the other fiber aggregate with the back-side waist portion 10b and the back-side body portion 20b using an adhesive.

In the production process described above, the left side 3a and right side 4a of the abdominal-side waist portion 10a and abdominal-side body portion 20a and the left side 3b and right side 4b of the back-side waist portion 10b and back-side body portion 20b are respectively joined on a portion following the body side when the diaper is worn, as illustrated in FIGS. 13A and 13B, but the diaper according to the embodiment is not limited to the diaper obtained by such production process. For example, in the diaper according to the embodiment, a width of the frontal area constituted by the abdominal-side waist portion 10a and abdominal-side body portion 20a and a width of the rear area constituted by the back-side waist portion 10b and the back-side body portion 20b are nearly the same in length, but the junction may be formed, for example, on the frontal side or on the rear side, by making the width of either the frontal side or the rear side longer for example. Moreover, one sheet of extensible laminate may be used extending across from the abdominal side to the back side and the junction may be formed either on the frontal side or on the rear side. Moreover, the frontal area and the rear area may be formed continuously into a cylindrical girth or both ends of a separately formed crotch portion 20c may be joined respectively with the frontal area and the rear area.

### EXAMPLES

The diaper of the present invention constituted of an extensible laminate having a trilayer structure is described more specifically below based on working examples, but the present invention is not limited to the working examples below.

### Extensible Laminate with Trilayer Structure

Fiber aggregate (wave-shaped): A fiber aggregate was prepared that was constituted from a core and sheath type composite fiber in which a core material made of thermoplastic polyurethane (TPU) was covered by a sheath material of polypropylene (PP). The area ratio of the sheath material to the core material was 10:90 in the cross-section of the composite fiber. The basis weight of the fiber aggregate was 30 g/m². The fiber aggregate was prepared so as to have a wave shape in which the pitch of the fiber aggregate (number of ridge portions 7b per 1 cm in the width direction) was 3.93 cm⁻¹, the difference between heights of lower ends of the valley portions 7a and upper ends of the ridge portions 7b was 1 mm, and the width of the ridge portions 7b was 1 mm. Fiber aggregate (flat): A fiber aggregate was formed in a flat manner using the same core and sheath type composite fiber as the wave-shaped fiber aggregate. The basis weight of the fiber aggregate was 15 g/m². Elastomeric molded body: A film production apparatus (model VS30, manufactured by Tanabe Plastic Machine) consisting of a T-die single-screw melt extruder and a chill roll was used to prepare strand-shaped elastomeric molded bodies having a circular cross-section with a diameter of 0.5 mm wherein a core material made of SIS copolymer was covered by a sheath material made of polypropylene (PP). The area ratio of the sheath material to the core material in the cross-section was 2:98. The strand-shaped elastomeric molded bodies were arranged so that there were 5 strands/cm in the width direction. The basis weight of the elastomeric molded bodies was 50 g/m². Joining of the fiber aggregate to the elastomeric molded bodies was done by laminating (thermal fusion bonding) at a temperature of 200°C or more and 300°C or less, thereby preparing an extensible laminate.

### Strain Test

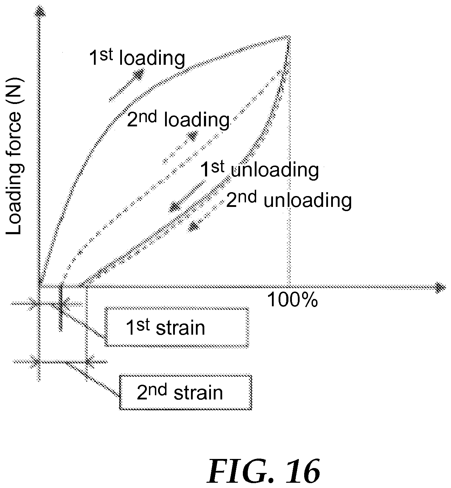

The extensible laminates obtained were formed with rectangular shapes of widths of 25 mm and lengths of 80 mm, and strain tests were performed. In the strain tests, the extensible laminate was gripped with a chucking distance of 25 mm, and a cycle of pulling at a pulling speed of 300 mm/min until the extension of the extensible laminate became 100% and then releasing the tension was repeated twice. FIG. 16 is a diagram showing the relationship between the stress generated in the extensible laminate and elongation during the strain test. As is shown in FIG. 16, the strain after the second cycle (the second strain) was measured, taking the first loading step and the first releasing step as a first cycle, and the second loading step and the second releasing step as the second cycle.

As a result of these measurements, the second strain was 15% of the length before the test. This value was sufficiently small compared to conventional extensible laminates.

### Bonding Strength (Delamination) Test

The extensible laminates obtained were formed with rectangular shapes of widths of 25 mm and lengths of 80 mm, and bonding strength (delamination) tests were performed. In the bonding strength test, the end portion of a trilayer extensible laminate was divided into a fiber aggregate (wave-form), a fiber aggregate (flat) and elastomeric molded bodies. The divided fiber aggregate (wave-form) and the fiber aggregate (flat) were each inserted into respective chucks. The distance between the chucks was 25 mm. An average value of the bonding strength of the extensible laminate (the load necessary for peeling off the extensible laminate) was measured at a pulling speed of 500 mm/min.

As a result of these measurements, the average bonding strength of the extensible laminate was 3.3 N/cm. These values were large compared to the conventional extensible laminates, and it was possible to realize sufficient durability even in the case of joints that did not use any adhesive materials and that were joined only by thermal fusion bonding.

## Claims

1. A diaper (101) comprising a waist opening portion (1) and a pair of leg opening portions (2a, 2b);
the diaper (101) further comprising an abdominal-side waist portion (10a) and a back-side waist portion (10b), forming at least a portion of the waist opening portion (1), and a body portion (20) including an abdominal-side body portion (20a), a back-side body portion (20b), and a crotch portion (20c) positioned between these portions that forms at least a portion of the leg opening portions (2a, 2b);
wherein in any portion of the diaper (101) except the crotch portion (20c):
the diaper comprises a laminate (5) including
an elastic fiber aggregate nonwoven layer (7) and
a plurality of elastomeric molded bodies (6) arranged at intervals; and
the elastomeric molded bodies (6) have regions that are joined with the elastic fiber aggregate nonwoven layer (7) and regions that are separated from the elastic fiber aggregate nonwoven layer (7), and wherein the laminate (5) generates a two stage elastic force,
wherein the elastomeric molded bodies (6) are joined to the elastic fiber aggregate nonwoven layer (7) by thermal fusion bonds, and
wherein the elastic fiber aggregate nonwoven layer (7) comprises elastically resilient composite fibers.

2. The diaper according to claim 1, wherein the elastomeric molded bodies (6) are strand-shaped or film-shaped elastomeric molded bodies.

3. The diaper according to claim 1 or claim 2, wherein the elastic fiber aggregate nonwoven layer (7) is constituted by the elastically resilient composite fibers.

4. The diaper according to any one of the preceding claims, wherein a layer (E2) having thermal fusion adhesiveness with a surface of the elastically resilient composite fibers is present in a portion of a surface of the elastomeric molded bodies (6).

## Patentansprüche

1. Windel (101), aufweisend einen Taillenöffnungsabschnitt (1) und ein Paar Beinöffnungsabschnitte (2a, 2b);
wobei die Windel (101) ferner einen bauchseitigen Taillenabschnitt (10a) und einen rückseitigen Taillenabschnitt (10b), die mindestens einen Abschnitt des Taillenöffnungsabschnitts (1) bilden, und einen Körperabschnitt (20) aufweist, der einen bauchseitigen Körperabschnitt (20a), einen rückseitigen Körperabschnitt (20b) und einen Schrittabschnitt (20c), der zwischen diesen Abschnitten angeordnet ist und mindestens einen Abschnitt der Beinöffnungsabschnitte (2a, 2b) bildet, aufweist;
wobei in jedem Abschnitt der Windel (101) mit Ausnahme des Schrittabschnitts (20c) gilt:
die Windel weist ein Laminat (5) auf, mit
einer elastischen Faserverbund-Vliesschicht (7) und
mehreren in Abständen angeordneten elastomeren Formkörpern (6); und
die elastomeren Formkörper (6) weisen mit der elastischen Faserverbund-Vliesschicht (7) verbundene Bereiche und von der elastischen Faserverbund-Vliesschicht (7) getrennte Bereiche auf, und wobei das Laminat (5) eine zweistufige elastische Kraft erzeugt,
wobei die elastomeren Formkörper (6) mit der elastischen Faserverbund-Vliesschicht (7) durch thermische Schmelzbindungen verbunden sind, und
wobei die elastische Faserverbund-Vliesschicht (7) elastisch nachgiebige Verbundfasern aufweist.

2. Windel nach Anspruch 1, wobei die elastomeren Formkörper (6) strangförmige oder folienförmige elastomere Formkörper sind.

3. Windel nach Anspruch 1 oder Anspruch 2, wobei die elastische Faserverbund-Vliesschicht (7) durch die elastisch nachgiebigen Verbundfasern gebildet wird.

4. Windel nach einem der vorstehenden Ansprüche, wobei eine Schicht (E2) mit thermischer Schmelzklebrigkeit mit einer Oberfläche der elastisch nachgiebigen Verbundfasern in einem Abschnitt einer Oberfläche der elastomeren Formkörper (6) vorhanden ist.

## Revendications

1. Couche pour bébés (101) comprenant une partie d'ouverture de taille (1) et une paire de parties d'ouverture de jambe (2a, 2b) ;
la couche pour bébés (101) comprenant en outre une partie de taille côté abdominal (10a) et une partie de taille côté arrière (10b), formant au moins une partie de la partie d'ouverture de taille (1), et une partie de corps (20) incluant une partie de corps côté abdominal (20a), une partie de corps côté arrière (20b), et une partie d'entrejambe (20c) positionnée entre ces parties, qui forme au moins une partie des parties d'ouverture de jambe (2a, 2b) ;
dans laquelle, dans n'importe quelle partie de la couche pour bébés (101) à l'exception de la partie d'entrejambe (20c) :
la couche pour bébés comprend un stratifié (5) incluant
une couche de non-tissé à agrégat de fibres élastiques (7) et
une pluralité de corps moulés élastomères (6) agencés à des intervalles ; et
les corps moulés élastomères (6) ont des régions qui sont jointes à la couche de non-tissé à agrégat de fibres élastiques (7) et des régions qui sont séparées de la couche de non-tissé à agrégat de fibres élastiques (7) et dans laquelle le stratifié (5) génère une force élastique à deux étapes,
dans laquelle les corps moulés élastomères (6) sont joints à la couche de non-tissé à agrégat de fibres élastiques (7) par des liaisons par fusion thermique, et
dans laquelle la couche de non-tissé à agrégat de fibres élastiques (7) comprend des fibres composites élastiquement résilientes.

2. Couche pour bébés selon la revendication 1, dans laquelle les corps moulés élastomères (6) sont des corps moulés élastomères en forme de brin ou en forme de film.

3. Couche pour bébés selon la revendication 1 ou la revendication 2, dans laquelle la couche de non-tissé à agrégat de fibres élastiques (7) est constituée des fibres composites élastiquement résilientes.

4. Couche pour bébés selon l'une quelconque des revendications précédentes, dans laquelle une couche (E2) ayant une adhésivité par fusion thermique avec une surface des fibres composites élastiquement résilientes est présente dans une partie d'une surface des corps moulés élastomères (6).
